# EUROPÄISCHE PATENTSCHRIFT

(11) **EP 1 723 907 B1**
(45) Veröffentlichungstag und Bekanntmachung des Hinweises auf die Patenterteilung: **14.05.2008**
(21) Anmeldenummer: 05011037.8
(22) Anmeldetag: 20.05.2005
(51) Int. Cl.: A61B 5/15

(54) **Lanzettensystem mit Sterilschutz**
Lancet device with sterile protection
Dispositif de lancettes avec protection stérile

(43) Veröffentlichungstag der Anmeldung: 22.11.2006
(73) Patentinhaber: F. Hoffmann-La Roche AG, 4070 Basel (CH); Roche Diagnostics GmbH, 68305 Mannheim (DE)
(72) Erfinder: List, Hans, 64754 Hesseneck-Kailbach (DE)
(74) Vertreter: Huhn, Michael

(56) Entgegenhaltungen:
- WO-A-01/66010
- WO-A-02/36010
- US-A1- 2003 050 573
- US-A1- 2004 260 325

## Beschreibung

Die vorliegende Erfindung betrifft ein Lanzettensystem enthaltend mindestens eine Lanzette mit einer Lanzettenspitze, die in einem Sterilschutz eingebettet ist. Insbesondere betrifft die Erfindung ein als Magazin dienendes Lanzettensystem für mindestens zwei Lanzetten Des Weiteren betrifft die Erfindung eine Stechhilfe mit mindestens einem erfindungsgemäßen Lanzettensystem.

Eine Entnahme von Körperflüssigkeiten, insbesondere von Blut, wird vor allem mit dem Ziel einer nachfolgenden Analyse durchgeführt, um eine Diagnose von Krankheiten oder die Überwachung des Stoffwechselzustandes eines Patienten zu ermöglichen. Insbesondere von Diabetikern wird eine solche Entnahme durchgeführt, um die Blutzuckerkonzentration zu bestimmen. Um für Diagnosezwecke eine Entnahme von nur geringen Mengen Blut vorzunehmen, werden üblicherweise sterile, scharfe Lanzetten verwendet, die zum Beispiel von Krankenhauspersonal oder dem Patienten selbst bei dem Patienten in die Fingerbeere oder auch andere Körperteile kurz eingestochen werden. Vor allem im Bereich des sogenannten "home-monitoring", wobei medizinische Laien selbst einfache Analysen des Blutes durchführen, werden Lanzettensysteme und dazu passende Geräte (sogenannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen.

Die Lanzettenspitze von zur Blutgewinnung verwendeten Lanzetten wird typischerweise im Voraus sterilisiert und durch einen Sterilschutz (zum Beispiel in Form einer Kappe oder Tasche) in einem sterilen Zustand gehalten, bevor sie für einen Stechvorgang verwendet wird, um sicherzustellen, dass die Lanzettenspitze nicht durch die Umgebung kontaminiert wird. Ferner werden häufig Vorkehrungen dafür getroffen, dass die Lanzettenspitze nach einem erfolgten Stechvorgang erneut abgeschirmt wird (gegebenenfalls durch dieselbe Kappe oder Tasche), so dass unbeabsichtigte Verletzungen und damit einhergehende Infektionen durch an der Lanzettenspitze anhaftendes Blut vermieden werden.

Bei Einzellanzetten kann beispielsweise ein Sterilschutz hergestellt werden, indem die Spitze der Lanzette in einem Arbeitsgang mit der Erzeugung des Lanzettenkörpers mit Kunststoff umspritzt wird. Vor Gebrauch entfernt der Anwender dieses Teil manuell, meist beim Einsetzen in eine Stechhilfe. Im Fall von magazinierten Lanzetten sind ähnliche Sterilschutzeinrichtungen gebräuchlich, zum Beispiel solche, bei denen die Lanzette nach hinten aus einem Sterilschutz herausgezogen wird, woraufhin der Sterilschutz per Federkraft aus dem Stichweg befördert wird. Hierfür sind relativ aufwendige Mechaniken erforderlich, insbesondere Federn, die in das Verbrauchsmittel integriert sind.

In WO 01/66010 wird die Umständlichkeit dieser Mechanik umgangen, indem der Sterilschutz einfach durchstochen wird. WO 01/66010 bezieht sich auf eine Lanzette enthaltend eine Lanzettennadel mit einer Spitze und einen Lanzettenkörper, der die Lanzettennadel zumindest im Bereich der Spitze vollständig umgibt. Der Lanzettenkörper besteht zumindest im Bereich der Spitze der Lanzettennadel aus einem elastischen Material, in das die Spitze der Lanzettennadel eingebettet ist. Ferner wird eine Lanzette beschrieben, die eine Lanzettennadel mit einer Spitze und einen Hohlkörper, der zumindest die Spitze der Lanzettennadel umgibt, enthält. Die Lanzettennadel ist im Bereich ihrer Spitze im Hohlkörper beweglich und der Hohlkörper besteht zumindest teilweise aus einem elastischen Material, das von der Spitze der Lanzettennadel beim Stechvorgang durchstoßen werden kann und das sich gegebenenfalls nach dem Zurückziehen der Spitze der Lanzettennadel in den Hohlkörper wieder verschließt. Nachteilig daran ist, dass der elastische Umschluss auf dem gesamten Stichweg Reibung verursacht. Daher müssen die Antriebe der verwendeten Stechhilfen entsprechend dimensioniert sein. Dieser Umschluss der gebrauchten Lanzettenspitze ist aber in vielen Fällen von magazinierten oder einzeln vorliegenden Lanzetten nicht nötig, weil die Lanzettenspitze zum Beispiel in einen starren Hohlkörper zurückgezogen wird und somit eine Gefahr der Kontamination der Umgebung und eine Verletzungsgefahr nicht gegeben sind.

US 5,304,192 A betrifft eine Lanzettenvorrichtung mit einer Lanzettennadel, deren Spitze durch eine entfernbare Kappe umschlossen ist. Die Kappe stellt die Sterilität der Lanzettenspitze bis zur Benutzung sicher. Sie ist über eine zerbrechliche Verbindung mit einem Bestandteil der Lanzettenvorrichtung verbunden und wird zum Entfernen von diesem Bestandteil manuell abgedreht.

US 5,554,166 bezieht sich auf eine Blutlanzettenvorrichtung mit einer Lanzettennadel, die im unbenutzten Zustand mit einer Sterilschutzkappe versehen ist. Die Sterilschutzkappe wird zum Entfernen so gedreht, dass sie an vorbestimmten Bruchstellen abbricht.

Das manuelle Abdrehen der Kappen zum Freigeben der Lanzettenspitze für einen Stechvorgang ist ein zu vermeidender Schritt in einem Verfahren zur Entnahme von Körperflüssigkeiten, da er die Handhabung solcher Lanzetten aufwendig gestaltet.

Die Aufgabe der vorliegenden Erfindung ist es, die Nachteile des Standes der Technik zu beseitigen. Insbesondere ist es die Aufgabe der vorliegenden Erfindung, Lanzettensysteme oder Lanzettenmagazine bereitzustellen, bei denen zumindest die Lanzettenspitze im unbenutzten Zustand bis unmittelbar vor der ersten Benutzung steril gehalten wird und die Lanzette nach dieser Benutzung so aufbewahrt wird, dass eine Kontamination der Umgebung und eine unbeabsichtigte Verletzung des Benutzers vermieden wird. Weiterhin ist es Aufgabe der vorliegenden Erfindung, eine möglichst geringe Reibung der Lanzettennadel, insbesondere der Lanzettenspitze, mit dem Sterilschutz bei dem Stechvorgang und beim Zurückziehen der Lanzettenspitze nach dem Stechvorgang zu gewährleisten, so dass der Antrieb einer verwendeten Stechhilfe schwächer als im Stand der Technik gewählt, der Verschleiß an allen beweglichen Teilen der Stechhilfe verringert und die Lebensdauer der Stechhilfe vergrößert werden kann.

Diese Aufgabe wird erfindungsgemäß gelöst durch ein Lanzettensystem enthaltend mindestens eine Lanzette mit einer Lanzettenspitze. Die Lanzettenspitze ist von einem Sterilschutz, der zumindest teilweise aus einem zu Sprödbruch neigenden Material besteht, umgeben, wobei der Sterilschutz an dem zu Sprödbruch neigenden Material Sollbruchstellen aufweist und bei einem Stechvorgang durch die Lanzettenspitze an den Sollbruchstellen zum Freigeben der Lanzettenspitze zerbrechlich ist.

Das erfindungsgemäße Lanzettensystem weist mindestens eine Lanzette mit einer Lanzettenspitze auf. Gemäß einer bevorzugten Ausführungsform der vorliegenden Erfindung umfasst das Lanzettensystem eine Vielzahl von Lanzetten. Dabei kann das Lanzettensystem als Magazin für Lanzetten in eine Stechhilfe eingesetzt werden.

Die Lanzettenspitze wird beim bestimmungsgemäßen Gebrauch des Lanzettensystems in Gewebe eingestochen, um den Ausfluss einer Körperflüssigkeit, insbesondere von Blut oder interstitieller Flüssigkeit, zu verursachen. Die Lanzettenspitze kann zum Beispiel rotationssymmetrisch ausgebildet sein. Es können auch ein oder mehrere Schliffe an der Lanzettenspitze angebracht sein. Die hierfür vorhandenen, zur Längsachse der Lanzettenspitze geneigten und zu der Spitze zulaufenden Kanten dienen beim Einstich als scharfe Schneide und gestalten den Stechvorgang in vorteilhafterweise schmerzärmer, als dies mit rotationssymmetrischen Lanzettenspitzen der Fall ist.

Der Sterilschutz des erfindungsgemäßen Lanzettensystems schirmt die Lanzettenspitze im unbenutzten Zustand keimdicht ab, so dass Keime bis unmittelbar vor der Benutzung des erfindungsgemäßen Lanzettensystems nicht an die Lanzettenspitze gelangen können. Nach einer geeigneten Sterilisierung bleibt die Lanzettenspitze in dem Sterilschutz über lange Zeit steril. Der Sterilschutz kann z.B. ein die Lanzettenspitze umgebender Hohlkörper sein. Die Lanzettenspitze kann auch in den Sterilschutz eingebettet sein, wobei der Sterilschutz z.B. durch ein Umspritzen der Lanzettenspitze mittels eines Spritzgussverfahrens hergestellt werden kann.

Der Sterilschutz besteht zumindest teilweise aus einem zu Sprödbruch neigenden Material. Ein solches Material hat die Eigenschaft, dass es bei Krafteinwirkung vor seinem Bruch im Wesentlichen keine bleibende Formveränderung zeigt. Die Streckgrenze des zu Sprödbruch neigenden Materials ist vorzugsweise ungefähr gleich seiner Bruchgrenze. Für eine Lanzettenspitze aus Stahl mit einer Streckgrenze von ca. 500 N/mm² sollte das zu Sprödbruch neigende Material vorzugsweise eine Streckgrenze aufweisen, die unter 50 N/mm² liegt. Vorzugsweise weist das zu Sprödbruch neigende Material eine hohe Sprödigkeit und eine geringe Festigkeit auf, so dass wenig Energie zum Zerbrechen des Sterilschutzes im Bereich der Sollbruchstellen erforderlich ist. Insbesondere sollte das zu Sprödbruch neigende Material weich relativ zu dem Material der Lanzettenspitze/Lanzettennadel sein, damit diese beim Zerbrechen des zu Sprödbruch neigenden Materials nicht beschädigt wird. Für eine Lanzettenspitze z.B. aus Stahl mit einer Vickers-Härte von ca. 250 sollte die Vickers-Härte des zu Sprödbruch neigenden Materials bevorzugt unter 25 liegen.

Das zu Sprödbruch neigende Material weist bei dem Lanzettensystem gemäß der vorliegenden Erfindung Sollbruchstellen auf, an denen das Material bei Krafteinwirkung zerbricht und so die Lanzettenspitze zur Durchführung eines Stechvorgangs freigibt. Diese erfindungsgemäße Gestaltung des Sterilschutzes des Lanzettensystems hat den Vorteil, dass der Sterilschutz aufgrund der Sollbruchstellen ohne große Krafteinwirkung durch Zerbrechen geöffnet werden kann und dass sich die Lanzettenspitze bei geöffnetem Sterilschutz weitgehend ohne Reibung (z.B. aus einem Hohlkörper oder einer Stechhilfe heraus) bewegen kann. Somit benötigt eine für den Stechvorgang verwendete Stechhilfe, in das die erfindungsgemäße Lanzettensystem aufgenommen wird, keinen starken Antrieb und sie weist einen geringen Verschleiß auf.

Erfindungsgemäß wird der Sterilschutz durch die Lanzettenspitze oder die gesamte Lanzettennadel zerbrochen. Es erfolgt dazu eine Krafteinwirkung der Lanzettennadel, insbesondere der Lanzettenspitze, auf das zu Sprödbruch neigende Material. Dies hat den Vorteil, dass der Sterilschutz nicht aufwendig manuell geöffnet und entfernt werden muss, sondern beim Durchführen des Stechvorgangs durch die Lanzettennadel automatisch zerbrochen wird.

Gemäß einer bevorzugten Ausführungsform der vorliegenden Erfindung ist das zu Sprödbruch neigende Material des Sterilschutzes ein einen Füllstoff enthaltendes Thermoplast, eine einen Füllstoff enthaltende Metallseife oder ein einen Füllstoff enthaltendes Wachs. Thermoplaste können im erweichten Zustand durch Pressen, Extrudieren, Spritzgießen oder andere Formgebungsverfahren zu Formteilen verarbeitet werden. Die Eigenschaften der Thermoplaste lassen sich unter anderem durch Zusätze von Füllstoffen variieren. Bei der vorliegenden Erfindung wird die Sprödigkeit des Thermoplasts, Wachses oder der Metallseife vorzugsweise mit Hilfe des Füllstoffs eingestellt, um ein zu Sprödbruch neigendes Material zu erhalten, aus dem der Sterilschutz oder ein Teil davon hergestellt werden kann. Der Schmelzpunkt des zu Sprödbruch neigenden Materials sollte vorzugsweise größer als 70°C betragen, damit eine Lagerung des Sterilschutzes ohne Probleme möglich ist.

Der Füllstoff wird vorzugsweise ausgewählt aus der Gruppe Talkum, Graphit und Molybdendisulfid. Das Wachs wird vorzugsweise ausgewählt aus der Gruppe Paraffine, Stearine, synthetische Wachse wie PE-Wachse und natürliche Wachse wie Bienenwachs. Metallseifen sind Salze der Metalle Al, Ba, Ca, Cd, Co, Cr, Cu, Fe, Mg, Mn, Ni, Pb, Sn, Sr und Zn mit höheren Fett-, Harz- oder Naphthensäuren (Stearate, Palmitate, Oleate, Linoleate, Laurate, ...). Die Metallseife ist bei der vorliegenden Erfindung vorzugsweise ausgewählt aus der Gruppe Aluminiumstearat, Aluminiumpalmitat, Calciumstearat, Calciumpalmitat, Magnesiumstearat, Magnesiumpalmitat, Zinkstearat oder Zinkpalmitat, besonders bevorzugt Aluminiumstearat (je nach Typ -mono-, -di- oder -tristearat), Calciumstearat oder Magnesiumstearat. Der Thermoplast wird vorzugsweise ausgewählt aus der Gruppe Polyethylen und Polypropylen. Für das zu Sprödbruch neigende Material sind insbesondere weiche Thermoplaste (zum Beispiel Polyethylen) geeignet, die mit einem hohen Anteil an Talkum gefüllt sind. Talkum ist ein weicher Werkstoff, der den Nadelschliff der Nadelspitze beim Zerbrechen des zu Sprödbruch neigenden Materials nicht beschädigt. Die Talkumpartikel sind dabei gut geeignet, um die innere Festigkeit des Thermoplasts, Wachses oder der Metallseife herabzusetzen, so dass ein viskoelastisches Verhalten in Richtung spröde verschoben wird, ohne dass die Härte des Kunststoffes, Wachses oder der Metallseife steigt. Der Anteil an Füllstoff in dem Thermoplast liegt vorzugsweise bei mehr als 20 Vol.-%, besonders bevorzugt zwischen 20 und 50 Vol.-%.

Gemäß einer bevorzugten Ausführungsform der vorliegenden Erfindung verlaufen die Sollbruchstellen parallel zu der Lanzettenspitze und erstrecken sich zumindest über einen Teil der Länge des Sterilschutzes. Der Sterilschutz kann beispielsweise eine zylindrische Kappe sein, die auf der Lanzettenspitze sitzt. Die Sollbruchstellen verlaufen vorzugsweise in Richtung entlang der Mantelfläche der zylindrischen Kappe. Sie können dabei entlang der gesamten Länge der Mantelfläche oder nur entlang eines Teils der Länge der Mantelfläche (im Bereich der Lanzettenspitze) verlaufen.

Gemäß einer bevorzugten Ausführungsform der vorliegenden Erfindung teilen die Sollbruchstellen Teilbereiche des Sterilschutzes ein, die nach einem Zerbrechen des Sterilschutzes an den Sollbruchstellen von der Lanzettenspitze abstehen. Vorzugsweise haften die abstehenden Teilbereiche des Sterilschutzes nach dem Zerbrechen an der Lanzettenspitze, so dass sie nicht unkontrolliert in dem Lanzettensystem herumfallen. Dabei kann der Sterilschutz zum Beispiel in dem Teil, in dem die Sollbruchstellen diese Teilbereiche einteilen, aus dem zu Sprödbruch neigenden Material und in dem sich daran anschließenden Teil aus einem plastisch verformbaren Material gefertigt sein, so dass die an den Sollbruchstellen spröde auseinandergebrochenen Bereiche von dem plastischen Material von der Lanzettenspitze abstehend gehalten werden. Der Sterilschutz kann aber auch vollständig aus dem zu Sprödbruch neigenden Material gefertigt sein, wobei die durch Sollbruchstellen eingeteilten Bereiche zwar beim Zerbrechen voneinander an den Sollbruchstellen getrennt werden, jedoch mit einem keine Sollbruchstellen aufweisenden Teil des Sterilschutzes verbunden bleiben und dabei mit einem (gegebenenfalls kleinen Winkel) von der Lanzettenspitze abstehen. Das Vorsehen solcher Bereiche, die nach dem Zerbrechen des Sterilschutzes von der Lanzettenspitze abstehen, hat den Vorteil, dass diese nach dem Freigeben der Lanzettenspitze nicht lose z.B. in einem Hohlkörper oder einer Stechhilfe herumfallen und die Bewegung der Lanzettenspitze stören können.

Gemäß einer bevorzugten Ausführungsform der vorliegenden Erfindung enthält das Lanzettensystem eine Lanzettennadel, die die Lanzettenspitze und einen Nadelkörper umfasst, wobei der Nadelkörper zumindest teilweise von einem Lanzettenkörper umgeben ist. Der Lanzettenkörper kann dabei fest mit dem Nadelkörper verbunden sein oder der Nadelkörper kann (zur Durchführung des Stechvorgangs) relativ zu dem Lanzettenkörper beweglich sein. Der Lanzettenkörper kann den Nadelkörper teilweise oder vollständig umgeben. Die Lanzettennadel des erfindungsgemäßen Lanzettensystems ist aus einem Material gefertigt, das ausreichend hart ist, um eine mechanische Beanspruchung während des Stechvorgangs, insbesondere während des Zerbrechens des zu Sprödbruch neigenden Materials, und während der Bearbeitungsschritte oder eventuell sonstige Beanspruchungen ohne Deformation zu überstehen. Weiterhin muss das Material so beschaffen sein, dass von der Lanzettennadel während des Stechvorgangs keine Partikel abbrechen oder sich ablösen. Schließlich muss das Lanzettennadelmaterial auch so bearbeitbar sein, dass die Lanzettenspitze ausreichend spitz und die Kanten der Lanzettenspitze gegebenenfalls ausreichend scharf geschliffen werden können. Gut geeignete Materialien für die Lanzettennadel sind vor allem Metalle und von diesen insbesondere Edelstähle. Es sind jedoch auch Nadeln aus Keramik oder Kunststoffen möglich.

Gemäß einer bevorzugten Ausführungsform der vorliegenden Erfindung umfasst das erfindungsgemäße Lanzettensystem einen Hohlkörper, der zumindest die Lanzettenspitze umgibt, wobei die Lanzettenspitze in dem Hohlkörper beweglich ist. Der Hohlkörper, der zumindest die Lanzettenspitze umgibt, dient als Schutz vor unbeabsichtigten Verletzungen eines Benutzers an der Lanzettenspitze, insbesondere nach einem erfolgten Stechvorgang, wenn die Lanzettenspitze wieder in den Hohlkörper zurückbewegt wird. Die Lanzettenspitze ist in dem Hohlkörper beweglich, damit sie für die Durchführung eines Stechvorgangs aus dem Hohlkörper herausbewegt werden kann. Vorzugsweise weist der Hohlkörper eine Austrittsöffnung auf, durch die die Lanzettenspitze bei einem Stechvorgang austreten kann, wobei die Lanzettenspitze nach dem Stechvorgang durch die Austrittsöffnung in den Hohlkörper zurückbewegt werden kann.

Gemäß einer bevorzugten Ausführungsform der vorliegenden Erfindung ist der Hohlkörper rohrförmig ausgebildet, wobei seine beiden Enden Öffnungen aufweisen, die offen oder verschlossen sein können. Beispielsweise können beide Enden mit durchstoßbaren Folien aus einem elastischen oder plastischen Material verschlossen sein, die beim Stechvorgang geöffnet werden können. Eine der beiden Öffnungen dient beim Stechvorgang als Austrittsöffnung für die Lanzettenspitze. Die andere Öffnung kann zum Einführen eines Betätigungsmittels (zum Beispiel eines Stößels oder Hakens) dienen, das zur Bewegung der Lanzettenspitze beim Stechvorgang vorgesehen ist.

Der Lanzettenkörper kann relativ zu einem Hohlkörper beweglich oder mit diesem fest verbunden sein. Im ersten Fall ist die Lanzettennadel vorzugsweise fest mit dem Lanzettenkörper verbunden, im zweiten Fall ist sie relativ zu dem Lanzettenkörper in dem Hohlkörper beweglich. Der Lanzettenkörper kann als Führungselement für die Lanzettennadel dienen.

In einer bevorzugten Ausführungsform umgibt ein Hohlkörper den Lanzettenkörper und die Lanzettenspitze vollständig, wobei der Lanzettenkörper und die Lanzettenspitze in dem Hohlkörper gemeinsam verschiebbar sind. Dabei sind der Lanzettenkörper und die Lanzettenspitze bevorzugt in Längsrichtung in Bezug auf die Lanzettenspitze verschiebbar.

Für das getrennte oder gemeinsame Bewegen von Lanzettenspitze und Lanzettenkörper können in einer Stechhilfe, in der das erfindungsgemäße Lanzettensystem zum Einsatz kommt, die geeigneten konstruktiven Maßnahmen (zum Beispiel Betätigungsmittel, Antriebs- oder Halterungselemente) vorgesehen werden.

Gemäß einer bevorzugten Ausführungsform der vorliegenden Erfindung ist der Sterilschutz nach einem Zerbrechen an den Sollbruchstellen auf der Lanzettennadel von der Lanzettenspitze weg verschiebbar. Dabei zerbricht der Sterilschutz im Bereich der Lanzettenspitze an seinen Sollbruchstellen, um die Lanzettenspitze für einen Stechvorgang freizugeben, bleibt jedoch in einem anderen Bereich unversehrt und mit den zerbrochenen Teilen verbunden und wird auf der Lanzettennadel von der Lanzettenspitze weg verschoben. Diese Gestaltung des erfindungsgemäßen Lanzettensystems hat den Vorteil, dass der Sterilschutz während des Stechvorgangs nicht lose z.B. in dem Hohlkörper oder der Stechhilfe herumfallen und den Stechvorgang stören kann.

Damit die Lanzettennadel beziehungsweise die Lanzettenspitze das Zerbrechen des Sterilschutzes an den Sollbruchstellen bewirken kann, muss sie eine Kraft auf das zu Sprödbruch neigende Material im Bereich der Sollbruchstellen ausüben. Eine Möglichkeit ist, dass der Sterilschutz an der Innenseite eines Hohlkörpers fixiert ist und die Lanzettennadel oder Lanzettenspitze durch das zu Sprödbruch neigende Material des Sterilschutzes hindurchgestoßen wird. Vorzugsweise ist hingegen die Lanzettenspitze mit dem Sterilschutz so in einem Körper (z.B. in einem Hohlkörper oder einer Stechhilfe) beweglich, dass der Sterilschutz zum Zerbrechen an den Sollbruchstellen gegen eine Fläche, insbesondere eine Innenfläche des Körpers (z.B. des Hohlkörpers oder der Stechhilfe) gestoßen und die Lanzettenspitze dabei durch den Sterilschutz hindurchgetrieben werden kann. Dabei wird der (zum Beispiel als zylindrische Kappe gestaltete) Sterilschutz mit der Lanzettenspitze bewegt, bis er auf die Fläche, insbesondere die Innenfläche des Hohlkörpers oder der Stechhilfe stößt und daran zerbricht und/oder von der Innenfläche abgebremst wird, so dass die Lanzettenspitze durch den Sterilschutz hindurchgetrieben wird. Die Lanzettenspitze kann dann durch eine Austrittsöffnung in dem Hohlkörper oder der Stechhilfe austreten, um den Stechvorgang durchzuführen.

Die Erfindung bezieht sich weiterhin auf ein als Magazin gestaltetes Lanzettensystem mit mindestens zwei Lanzetten, die jeweils eine Lanzettenspitze aufweisen und jeweils in einzelnen, voneinander unabhängigen Kammern des Lanzettensystems enthalten sind, wobei die Lanzettenspitze in der Kammer beweglich ist. Die Lanzettenspitze ist dabei in der Kammer von einem Sterilschutz, der zumindest teilweise aus einem zu Sprödbruch neigenden Material besteht, umgeben, wobei der Sterilschutz Sollbruchstellen aufweist und bei einem Stechvorgang durch die Lanzettenspitze an den Sollbruchstellen zum Freigeben der Lanzettenspitze zerbrechlich ist.

Das als Magazin vorgesehene Lanzettensystem dient zur Aufbewahrung unbenutzter und benutzter Lanzetten. Die Kammern des Magazins übernehmen die Funktion des ggf. vorhandenen Hohlkörpers des oben beschriebenen, erfindungsgemäßen Lanzettensystems. Die Kammern sind vorzugsweise in dem Magazin regelmäßig geometrisch angeordnet, wobei benachbarte Kammern gemeinsame Wände aufweisen können. Das Magazin kann zum Beispiel stapel-, scheiben- oder trommelförmig aufgebaut sein.

Bis unmittelbar vor der Benutzung werden die Lanzettenspitzen in ihrem jeweiligen Sterilschutz steril aufbewahrt. Bei einem Stechvorgang wird das zu Sprödbruch neigende Material des Sterilschutzes mit Hilfe der Lanzettennadel, insbesondere mit Hilfe der Lanzettenspitze, an den Sollbruchstellen zerbrochen, so dass die Lanzettenspitze für den Stechvorgang freigegeben wird. Dazu tritt die Lanzettenspitze durch eine Austrittsöffnung aus ihrer Kammer aus. Nach dem Stechvorgang wird die Lanzettenspitze wieder durch die Austrittsöffnung in ihre Kammer zurückbewegt, so dass ein unbeabsichtigtes Verletzen an der Lanzettenspitze und eine Kontamination der Umgebung ausgeschlossen werden. Die sich durch das als Magazin dienende, erfindungsgemäße Lanzettensystem mit mindestens zwei Lanzetten ergebenden Vorteile entsprechen unter anderem den bereits für das erfindungsgemäße Lanzettensystem genannten.

Die Erfindung bezieht sich weiterhin auf eine Stechhilfe enthaltend mindestens einem erfindungsgemäßen Lanzettensystem und ein Betätigungsmittel, das auf das Lanzettensystem zur Bewegung der Lanzettenspitze z.B. in dem Hohlkörper, der Stechhilfe oder der Kammer einwirken kann, so dass die Lanzettenspitze zur Durchführung des Stechvorgangs den Sterilschutz an den Sollbruchstellen zerbrechen und aus dem Hohlkörper, der Stechhilfe oder der Kammer austreten kann. In die Stechhilfe werden die erfindungsgemäßen Lanzettensysteme einzeln manuell durch den Benutzer eingelegt. Es kann z.B. eine Vielzahl von Lanzetten (aufbewahrt in einem als Magazin dienenden, erfindungsgemäßen Lanzettensystem) in der Stechhilfe bereitgestellt werden. Ein in der Stechhilfe enthaltenes Betätigungsmittel (zum Beispiel eine Art Stößel oder Haken) wirkt in der Stechhilfe auf eine Lanzette, um die Lanzettenspitze zum Zerbrechen des zu Sprödbruch neigenden Materials an den Sollbruchstellen und zur Ausführung des Stechvorgangs zu bewegen. Das Betätigungsmittel kann ferner dazu dienen, die Lanzettenspitze nach einem Stechvorgang in ihren Hohlkörper, in die Stechhilfe beziehungsweise in ihre Kammer zurückzubewegen. Es kann zu diesem Zweck jedoch auch ein weiteres Element (zum Beispiel eine Feder) in der Stechhilfe vorgesehen sein.

Die Erfindung wird anhand der Zeichnung nachstehend näher erläutert.
- Figur 1: zeigt schematisch ein erfindungsgemäßes Lanzettensystem im Schnitt und den Ablauf eines Stechvorgangs mit diesem Lanzettensystem.
- Figur 2: zeigt den Lanzettenkörper, die Lanzettennadel und den Sterilschutz eines Lanzettensystems gemäß Figur 1.

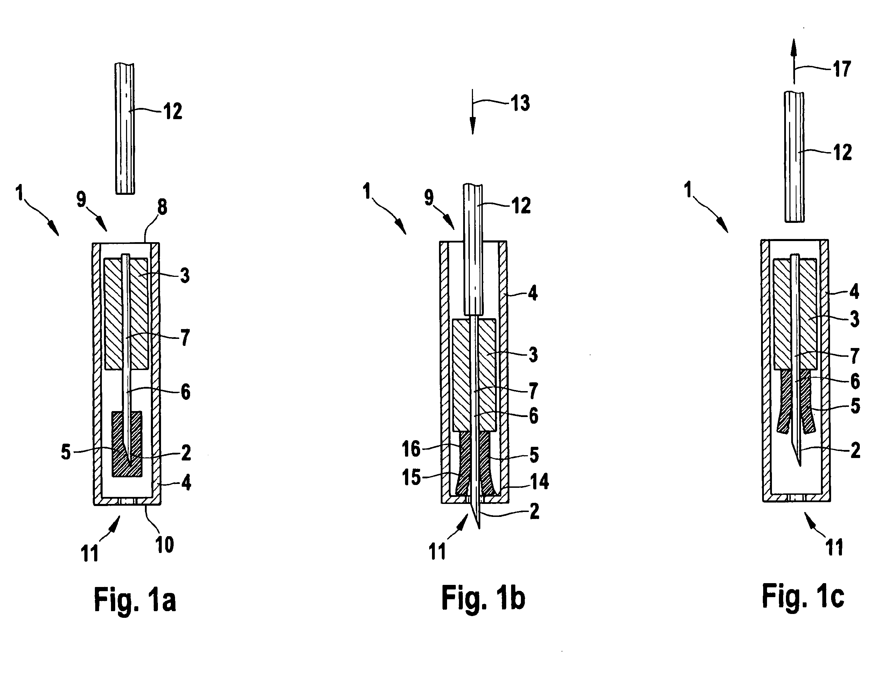

Das Lanzettensystem 1 aus Figur 1 weist eine Lanzettenspitze 2, einen Lanzettenkörper 3 und einen Hohlkörper 4 auf. In Figur 1a) ist das Lanzettensystem 1 vor dem Gebrauch dargestellt. Die Lanzettenspitze 2 ist in dem Hohlkörper 4 von einem Sterilschutz 5 keimdicht umgeben, der zumindest teilweise aus einem zu Sprödbruch neigenden Material besteht. Der Sterilschutz 5 ist eine auf der Lanzettenspitze 2 sitzende zylindrische Kappe, die (in Figur 1 nicht dargestellte) Sollbruchstellen aufweist.

Das Lanzettensystem 1 enthält eine Lanzettennadel 6, die die Lanzettenspitze 2 und einen Nadelkörper 7 umfasst, wobei der Nadelkörper 7 teilweise von dem Lanzettenkörper 3 umgeben ist. Der Lanzettenkörper 3 dient als Führungselement für die in dem Hohlkörper 4 beweglich angeordnete Lanzettennadel 6. Der Hohlkörper 4 ist rohrförmig ausgebildet mit einer an dem der Lanzettenspitze 2 abgewandten ersten Ende 8 ausgebildeten ersten Öffnung 9 und einer an dem der Lanzettenspitze 2 zugewandeten zweiten Ende 10 ausgebildeten zweiten Öffnung 11. Der Hohlkörper 4 umgibt den Lanzettenkörper 3, die Lanzettennadel 6 und den Sterilschutz 5 vollständig, wobei diese drei Elemente gemeinsam in dem Hohlkörper 4 in Längsrichtung verschiebbar sind. Ein Betätigungsmittel 12 ist zum Beispiel in der erfindungsgemäßen Stechhilfe vorgesehen, um das Lanzettensystem 1 zur Durchführung eines Stechvorgangs zu betätigen.

Figur 1b) zeigt einen mit dem erfindungsgemäßen Lanzettensystem ausgeführten Stechvorgang. Das Betätigungsmittel 12 wird dazu durch die erste Öffnung 9 in den Hohlkörper 4 bewegt. Das Betätigungsmittel 12 übt eine Kraft in Stechrichtung 13 auf Lanzettenkörper 3, Lanzettennadel 6 und Sterilschutz 5 aus. Dadurch bewegen sich diese Elemente des Lanzettensystems 1 auf die zweite Öffnung 11 des Hohlkörpers 4 zu, bis der Sterilschutz 5 auf die die zweite Öffnung 11 umgebende Innenfläche 14 des Hohlkörpers 4 stößt und die Lanzettenspitze 2 durch den Sterilschutz 5 hindurchgetrieben wird. Dabei zerbricht das zu Sprödbruch neigende Material des Sterilschutzes 5 an den Sollbruchstellen und gibt die Lanzettenspitze 2 frei. Diese tritt durch die als Austrittsöffnung dienende zweite Öffnung 11 aus dem Hohlkörper 4 aus und erzeugt einen Einstich zum Beispiel in dem Finger eines Benutzers. Der Sterilschutz 5 wird dabei auf der Lanzettennadel 6 von der Lanzettenspitze 2 weg verschoben. Im Bereich der Sollbruchstellen 15 ist er zerbrochen, in einem Bereich ohne Sollbruchstellen 16 ist er intakt und umgibt weiterhin die Lanzettennadel 6, wobei er auf dieser verschiebbar ist. Somit befinden sich keine losen Bruchstücke des Sterilschutzes 5 in dem Hohlkörper 4.

Figur 1c) zeigt die in den Hohlkörper 4 nach dem Stechvorgang durch die Austrittsöffnung 11 zurückgezogene Lanzettenspitze 2. Das Zurückziehen kann zum Beispiel durch das Betätigungsmittel 12 bewirkt werden, das sich an den Lanzettenkörper 3 anheftet (zum Beispiel mit diesem verhakt) und in Rückzugsrichtung 17 zieht. Durch den Hohlkörper 4, der die Lanzettenspitze 2 nun erneut schützend umgibt, wird eine Verletzungsgefahr durch die benutzte Lanzettenspitze 2 vermieden, ebenso wie eine Kontamination der Umgebung durch an der Lanzettenspitze 2 haftende Körperflüssigkeitsreste.

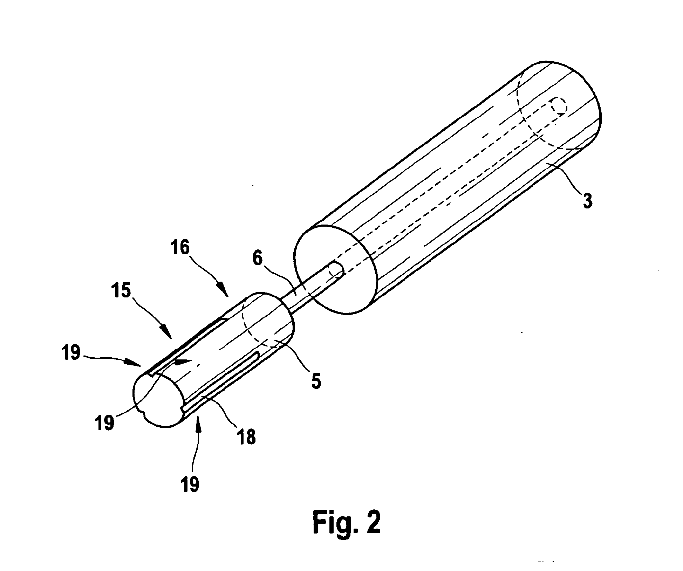

Figur 2 zeigt den Lanzettenkörper 3, die Lanzettennadel 6 und den Sterilschutz 5 des Lanzettensystems 1 aus Figur 1 vor der Benutzung (Figur 1a)). Zur Veranschaulichung sind diese Elemente des Lanzettensystems 1 ohne den sie umgebenden Hohlkörper 4 dargestellt. Der Sterilschutz 5 umgibt die (nicht dargestellte) Lanzettenspitze 2 keimdicht. Er weist Sollbruchstellen 18 auf, die parallel zu der Lanzettenspitze 2 verlaufen und sich nur über ca. 2/3 der Länge des Sterilschutzes 5 erstrecken. Zumindest der Bereich 15 des Sterilschutzes 5 mit den Sollbruchstellen 18 besteht aus einem zu Sprödbruch neigenden Material, insbesondere aus mit einem hohen Anteil an Talkum gefülltem Polyethylen. Die Sollbruchstellen 18 teilen Teilbereiche 19 des Sterilschutzes 5 ein, die nach einem Zerbrechen des Sterilschutzes 5 an den Sollbruchstellen 18 von der Lanzettenspitze 2 abstehen (siehe Figur 1b)). Der Bereich 16 des Sterilschutzes 5 ohne Sollbruchstellen zerbricht dabei nicht, sondern umgibt weiterhin die Lanzettennadel 6, auf der der Sterilschutz 5 dann von der Lanzettenspitze 2 weg in Richtung Lanzettenkörper 3 verschoben werden kann (siehe Figur 1b)).

### Bezugszeichenliste

- 1: Lanzettensystem
- 2: Lanzettenspitze
- 3: Lanzettenkörper
- 4: Hohlkörper
- 5: Sterilschutz
- 6: Lanzettennadel
- 7: Nadelkörper
- 8: erstes Ende des Hohlkörpers
- 9: erste Öffnung
- 10: zweites Ende des Hohlkörpers
- 11: zweite Öffnung, Austrittsöffnung
- 12: Betätigungsmittel
- 13: Stechrichtung
- 14: Innenfläche
- 15: Bereich des Sterilschutzes mit Sollbruchstellen
- 16: Bereich des Sterilschutzes ohne Sollbruchstellen
- 17: Rückzugsrichtung
- 18: Sollbruchstellen
- 19: Teilbereiche

## Patentansprüche

1. Lanzettensystem (1) enthaltend mindestens eine Lanzette mit einer Lanzettenspitze (2), **dadurch gekennzeichnet, dass** die Lanzettenspitze (2) von einem Sterilschutz (5), der zumindest teilweise aus einem zu Sprödbruch neigenden Material besteht, umgeben ist, wobei der Sterilschutz (5) an dem zu Sprödbruch neigenden Material Sollbruchstellen (18) aufweist und bei einem Stechvorgang durch die Lanzettenspitze (2) an den Sollbruchstellen (18) zum Freigeben der Lanzettenspitze (2) zerbrechlich ist.

2. Lanzettensystem gemäß Anspruch 1, **gekennzeichnet durch** einen Hohlkörper (4), der die Lanzettenspitze (2) umgibt, wobei die Lanzettenspitze (2) in dem Hohlkörper (4) beweglich ist

3. Lanzettensystem gemäß Anspruch 2, **dadurch gekennzeichnet, dass** der Hohlkörper (4) eine Austrittsöffnung (11) aufweist, durch die die Lanzettenspitze (2) bei einem Stechvorgang austreten kann, wobei die Lanzettenspitze (2) nach dem Stechvorgang durch die Austrittsöffnung (11) in den Hohlkörper (4) zurückbewegt werden kann.

4. Lanzettensystem gemäß einem der Ansprüche 1 bis 3, **dadurch gekennzeichnet, dass** die Lanzettenspitze (2) so in dem Hohlkörper (4) oder in einem anderen Körper beweglich ist, dass der Sterilschutz (5) zum Zerbrechen an den Sollbruchstellen (18) gegen eine Innenfläche (14) des Hohlkörpers (4) oder des anderen Körpers gestoßen und die Lanzettenspitze (2) dabei durch den Sterilschutz (5) hindurchgetrieben werden kann.

5. Lanzettensystem gemäß einem der Ansprüche 1 bis 4, **dadurch gekennzeichnet, dass** das zu Sprödbruch neigende Material des Sterilschutzes (5) ein(e) einen Füllstoff enthaltende(s) Thermoplast, Wachs oder Metallseife ist.

6. Lanzettensystem gemäß Anspruch 5, **dadurch gekennzeichnet, dass** der Füllstoff mindestens ein Stoff ausgewählt aus der Gruppe Talkum, Graphit und Molybdendisulfid ist.

7. Lanzettensystem gemäß einem der Ansprüche 5 oder 6, **dadurch gekennzeichnet, dass** der Thermoplast ein Thermoplast ausgewählt aus der Gruppe Polyethylen oder Polypropylen ist.

8. Lanzettensystem gemäß einem der Ansprüche 1 bis 7, **dadurch gekennzeichnet, dass** die Sollbruchstellen (18) parallel zu der Lanzettenspitze (2) verlaufen und sich zumindest über einen Teil der Länge des Sterilschutzes (5) erstrecken.

9. Lanzettensystem gemäß einem der Ansprüche 1 bis 8, **dadurch gekennzeichnet, dass** die Sollbruchstellen (18) Teilbereiche (19) des Sterilschutzes (5) einteilen, die nach einem Zerbrechen des Sterilschutzes (5) an den Sollbruchstellen (18) von der Lanzettenspitze (2) abstehen.

10. Lanzettensystem gemäß einem der Ansprüche 1 bis 9, **dadurch gekennzeichnet, dass** der Sterilschutz (5) eine auf der Lanzettenspitze (2) sitzende zylindrische Kappe ist.

11. Lanzettensystem gemäß einem der Ansprüche 1 bis 10, enthaltend eine Lanzettennadel (6), die die Lanzettenspitze (2) und einen Nadelkörper (7) umfasst, wobei der Nadelkörper (7) zumindest teilweise von einem Lanzettenkörper (3) umgeben ist.

12. Lanzettensystem gemäß Anspruch 11, **dadurch gekennzeichnet, dass** der Lanzettenkörper (3) als Führungselement für die Lanzettennadel (6) dient.

13. Lanzettensystem gemäß einem der Ansprüche 11 oder 12, **dadurch gekennzeichnet, dass** der Sterilschutz (5) nach einem Zerbrechen an den Sollbruchstellen (18) auf der Lanzettennadel (6) von der Lanzettenspitze (2) weg verschiebbar ist.

14. Lanzettensystem mit mindestens zwei Lanzetten (1), die jeweils eine Lanzettenspitze (2) aufweisen und jeweils in einzelnen, voneinander unabhängigen Kammern des Lanzettensystems enthalten sind, wobei die Lanzettenspitze (2) in der Kammer beweglich ist, **dadurch gekennzeichnet, dass** die Lanzettenspitze (2) in der Kammer von einem Sterilschutz (5), der zumindest teilweise aus einem zu Sprödbruch neigenden Material besteht, umgeben ist, wobei der Sterilschutz (5) Sollbruchstellen (18) aufweist und bei einem Stechvorgang durch die Lanzettenspitze (2) an den Sollbruchstellen (18) zum Freigeben der Lanzettenspitze (2) zerbrechlich ist.

15. Stechhilfe enthaltend mindestens ein Lanzettensystem (1) gemäß einem der Ansprüche 1 bis 14 und ein Betätigungsmittel (12), das auf das Lanzettensystem (1) zur Bewegung der Lanzettenspitze (2) einwirken kann, so dass die Lanzettenspitze (2) zur Durchführung des Stechvorgangs den Sterilschutz (5) an den Sollbruchstellen (18) zerbrechen kann.

## Claims

1. Lancet system (1) containing at least one lancet with a lancet tip (2), **characterised in that** the lancet tip (2) is surrounded by a sterile protective sheath (5), which consists at least in part of a material that tends to undergo brittle fracture, wherein the sterile protective sheath (5) comprises desired fracture sites (18) on the material tending to undergo brittle fracture and in a lancing procedure can be broken by the lancet tip (2) at the desired fracture sites (18) to free the said lancet tip (2).

2. Lancet system according to claim 1, **characterised by** a hollow body (4) that surrounds the lancet tip (2), wherein the lancet tip (2) is movable in the hollow body (4).

3. Lancet system according to claim 2, **characterised in that** the hollow body (4) comprises an outlet opening (11) through which the lancet tip (2) can emerge in a lancing procedure, wherein the lancet tip (2) can be retracted through the outlet opening (11) into the hollow body (4) after the lancing procedure.

4. Lancet system according to one of claims 1 to 3, **characterised in that** the lancet tip (2) is movable in the hollow body (4) or in another body so that the sterile protective sheath (5) can impact against an internal surface (14) of the hollow body (4) or of the other body so as to rupture at the desired fracture sites (18), and the lancet tip (2) can thereby be passed through the sterile protective sheath (5).

5. Lancet system according to one of claims 1 to 4, **characterised in that** the material of the sterile protective sheath (5) tending to undergo brittle fracture is a thermoplastic material, wax or metal soap containing a filler.

6. Lancet system according to claim 5, **characterised in that** the filler is at least one substance selected from the group comprising talcum, graphite and molybdenum disulfide.

7. Lancet system according to one of claims 5 or 6, **characterised in that** the thermoplastic material is selected from the group comprising polyethylene or polypropylene.

8. Lancet system according to one of claims 1 to 7, **characterised in that** the desired fracture sites (18) run parallel to the lancet tip (2) and extend at least over part of the length of the sterile protective sheath (5).

9. Lancet system according to one of claims 1 to 8, **characterised in that** the desired fracture sites (18) separate partial regions (19) of the sterile protective sheath (5), which after a breakage of the sterile protective sheath (5) at the desired fracture sites (18) project from the lancet tip (2).

10. Lancet system according to one of claims 1 to 9, **characterised in that** the sterile protective sheath (5) is a cylindrical cap sitting on the lancet tip (2).

11. Lancet system according to one of claims 1 to 10 containing a lancet needle (6), which includes the lancet tip (2) and a needle body (7), wherein the needle body (7) is surrounded at least in part by a lancet body (3).

12. Lancet system according to claim 11, **characterised in that** the lancet body (3) serves as a guide element for the lancet needle (6).

13. Lancet system according to one of claims 11 or 12, **characterised in that** the sterile protective sheath (5) is displaceable away from the lancet tip (2) after a breakage at the desired fracture sites (18) on the lancet needle (6).

14. Lancet system with at least two lancets (1), each of which comprises a lancet tip (2) and each of which is contained in individual chambers of the lancet system independent of one another, the lancet tip (2) being movable in the chamber, **characterised in that** the lancet tip (2) is surrounded in the chamber by a sterile protective sheath (5) which consists at least in part of a material tending to undergo brittle fracture, wherein the sterile protective sheath (5) comprises desired fracture sites (18) and in a lancing procedure can be broken by the lancet tip (2) at the desired fracture sites (18) to free the lancet tip (2).

15. Lancing aid containing at least one lancet system (1) according to one of claims 1 to 14 and an actuating means (12) that can act on the lancet system (1) to move the lancet tip (2), so that the lancet tip (2) can break the sterile protective sheath (5) at the desired fracture sites (18) to allow the implementation of the lancing procedure.

## Revendications

1. Système de lancettes (1) contenant au moins une lancette comprenant une pointe de lancette (2), **caractérisé en ce que** la pointe de lancette (2) est entourée d'une protection stérile (5) qui est constituée au moins en partie d'une matière manifestant une tendance à la rupture de fragilité, la protection stérile (5) présentant des zones destinées à la rupture (18) sur la matière manifestant une tendance à la rupture de fragilité et, lors d'un processus de piqûre via la pointe de lancette (2), possédant une aptitude à la rupture aux zones destinées à la rupture (18) pour la libération de la pointe de lancette (2).

2. Système de lancettes selon la revendication 1, **caractérisé par** un corps creux (4) qui entoure la pointe de lancette (2), la pointe de lancette (2) étant mobile dans le corps creux (4).

3. Système de lancettes selon la revendication 2, **caractérisé en ce que** le corps creux (4) présente une ouverture de sortie (11) à travers laquelle, dans un processus de piqûre, la pointe de lancette (2) peut sortir, la pointe de lancette (2) étant à même de retourner, après le processus de piqûre, à travers l'ouverture de sortie (11), dans le corps creux (4).

4. Système de lancettes selon l'une quelconque des revendications 1 à 3, **caractérisé en ce que** la pointe de lancette (2) est mobile dans le corps creux (4) ou dans un autre corps de telle sorte que la protection stérile (5) pour être rompue aux zones destinées à la rupture (18) et poussée contre une surface interne (14) du corps creux (4) ou de l'autre corps, la pointe de lancette (2) étant ainsi à même de passer à travers la protection stérile (5).

5. Système de lancettes selon l'une quelconque des revendications 1 à 4, **caractérisé en ce que** la matière de la protection stérile (5), manifestant une tendance à la rupture de fragilité, est une matière thermoplastique, de la cire ou du savon métallique qui contient une matière de charge.

6. Système de lancettes selon la revendication 5, **caractérisé en ce que** la matière de charge représente au moins une substance choisie parmi le groupe du talc, du graphite et du disulfure de molybdène.

7. Système de lancettes selon l'une quelconque des revendications 5 ou 6, **caractérisé en ce que** la matière thermoplastique est une matière thermoplastique choisie parmi le groupe du polyéthylène ou du polypropylène.

8. Système de lancettes selon l'une quelconque des revendications 1 à 7, **caractérisé en ce que** les zones destinées à la rupture (18) sont disposées parallèlement à la pointe de lancette (2) et s'étendent au moins sur une partie de la longueur de la protection stérile (5).

9. Système de lancettes selon l'une quelconque des revendications 1 à 8, **caractérisé en ce que** les zones destinées à la rupture (18) répartissent la protection stérile (5) en zones partielles (19) qui, après une rupture de la protection stérile (5) aux zones destinées à la rupture (18), s'écartent de la pointe de lancette (2).

10. Système de lancettes selon l'une quelconque des revendications 1 à 9, **caractérisé en ce que** la protection stérile (5) est un capuchon cylindrique qui vient s'appliquer sur la pointe de lancette (2).

11. Système de lancettes selon l'une quelconque des revendications 1 à 10, contenant une aiguille de lancette (6) qui comprend la pointe de lancette (2) et un corps d'aiguille (7), le corps d'aiguille (7) étant entouré au moins en partie par un corps de lancette (3).

12. Système de lancettes selon la revendication 11, **caractérisé en ce que** le corps de lancette (3) fait office d'élément de guidage pour l'aiguille de lancette (6).

13. Système de lancettes selon l'une quelconque des revendications 11 ou 12, **caractérisé en ce que** la protection stérile (5), après une rupture aux zones destinées à la rupture (18) sur l'aiguille de lancette (6), est à même de s'écarter de la pointe de lancette (2).

14. Système de lancettes comprenant au moins deux lancettes (1) qui présentent respectivement une pointe de lancette (2) et qui sont contenues respectivement dans des chambres individuelles, indépendantes l'une de l'autre, du système de lancettes, la pointe de lancette (2) étant mobile dans la chambre, **caractérisé en ce que** la pointe de lancette (2), dans la chambre, est entourée d'une protection stérile (5) qui est constituée au moins en partie d'une matière manifestant une tendance à la rupture de fragilité, la protection stérile (5) présentant des zones destinées à la rupture (18) et, lors d'un processus de piqûre via la pointe de lancette (2), possédant une aptitude à la rupture aux zones destinées à la rupture (18) pour la libération de la pointe de lancette (2).

15. Auxiliaire de piqûre contenant au moins un système de lancettes (1) selon l'une quelconque des revendications 1 à 14 et un moyen d'actionnement (12) qui peut agir sur le système de lancettes (1) afin de mobiliser la pointe de lancette (2), si bien que la pointe de lancette (2) est à même de rompre, pour la mise en oeuvre du processus de piqûre, la protection stérile (5) aux zones destinées à la rupture (18).
